# EUROPÄISCHE PATENTSCHRIFT

(11) **EP 1 336 375 B1**
(45) Veröffentlichungstag und Bekanntmachung des Hinweises auf die Patenterteilung: **05.05.2010**
(21) Anmeldenummer: 03003032.4
(22) Anmeldetag: 12.02.2003
(51) Int. Cl.: A61B 5/15

(54) **System zur schmerzarmen Blutentnahme**
System for painless blood sampling
Dispositif pour le prélèvement indolore de sang

(30) Priorität: 15.02.2002 DE 10206254
(43) Veröffentlichungstag der Anmeldung: 20.08.2003
(73) Patentinhaber: Roche Diagnostics GmbH, 68305 Mannheim (DE); F. Hoffmann-La Roche AG, 4070 Basel (CH)
(72) Erfinder: Garthe, Claus-Dieter, 68623 Lampertheim-Neuschloss (DE); Ruschke, Peter, 55257 Budenheim (DE); Schmelzeisen-Redeker, Guenther, 64653 Lorsch (DE); Immekus, Claudio, 79312 Emmendingen (DE); Wolf, Uschi, 64295 Darmstadt (DE); Masuch, Klemens, 68642 Buerstadt (DE); List, Hans, 64754 Hesseneck-Kailbach (DE)

(56) Entgegenhaltungen:
- US-A- 6 045 567
- US-B1- 6 306 152

## Beschreibung

Die vorliegende Erfindung betrifft ein System zur schmerzfreien Blutentnahme, welches ein Lanzettensystem umfaßt, mit dem eine kleine Hautöffnung erzeugt wird, aus der Blut austreten kann, sowie einen Impulsgeber, der dazu dient, das Schmerzempfinden bei der Blutentnahme zu verringern.

Systeme zur Blutentnahme werden vorwiegend von Diabetikern benutzt, um den Blutzuckerspiegel zu kontrollieren. Derartige Blutentnahmegeräte werden jedoch auch in Arztpraxen, Krankenhäusern usw. eingesetzt, sofern für eine Untersuchung bzw. einen analytischen Test lediglich kleine Blutmengen im Bereich weniger µl oder darunter benötigt werden. Aufgrund der Notwendigkeit einer häufigen Testung haben sich die Blutentnahmegeräte jedoch eine besondere Bedeutung unter Diabetikern erworben. Hier ist eine ständige, häufig täglich mehrfache Blutentnahme notwendig, um zu hohe oder auch zu niedrige Blutzuckerwerte zu vermeiden und so Spätfolgen zu reduzieren. Während eine Kontrolle des Blutzuckerspiegels für Typ 1 Diabetiker nahezu unerläßlich ist und der von einer Vielzahl der Betroffenen auch durchgeführt wird, gehen eine Vielzahl von Typ 2 Diabetikern, der sogenannten Altersdiabetes, wesentlich unachtsamer mit ihrer Erkrankung um und führen keine oder nur unregelmäßige Blutzuckermessungen durch. Ein wesentlicher Grund für dieses Verhalten liegt in der für eine Analyse notwendigen Blutentnahme, die als schmerzhaft oder zumindest als unangenehm empfunden wird. Es ist daher ein wichtiges Ziel, den für eine Blutentnahme auftretenden Schmerz weitestgehend zu reduzieren, um Diabetikern das Leben zu erleichtern als auch um eine größere Zahl von Typ 2 Diabetikern zur Blutzuckermessung zu bewegen. So kann sowohl ein persönlicher als auch ein gesamtwirtschaftlicher Nutzen durch Vermeidung von Folgeerkrankungen erzielt werden.

Im Stand der Technik hat es bereits einige Bestrebungen gegeben, den beim Einstich auftretenden Schmerz zu verringern. Einen nicht unerheblichen Einfluß auf den ausgelösten Schmerz hat die Form der verwendeten Lanzettennadel, so daß durch eine Optimierung des Schliffes der Lanzettennadel bereits eine gewisse Schmerzreduktion erzielt werden kann. Weiterhin hat die Art der Stichbewegung einen Einfluß auf die Höhe des Schmerzes. Bei herkömmlichen Systemen, bei denen die Lanzette durch eine Feder auf einen Anschlag beschleunigt wird, tritt während des Stiches eine Vibration der Nadel auf, die Schmerz erzeugt. In dem europäischen Patent 0 565 970 wird eine verbesserte Blutlanzettenvorrichtung beschrieben, bei der die Lanzette durch einen Antrieb kontinuierlich eingestochen und auch aus der Wunde herausgezogen wird, so daß eine unregelmäßige, vibrierende Bewegung der Nadelspitze vermieden wird.

In der WO 01/62150 wird eine Blutlanzettenvorrichtung beschrieben, die auf einer Kappe, welche zur Blutentnahme an die Haut angedrückt wird, halbkugelförmige Erhöhungen aufweist, die die Nerven, welche den Schmerz leiten, verwirren sollen, so daß der Einstich mit der Lanzette vom Benutzer als weniger schmerzhaft empfunden wird.

In der US 6,306,152 wird eine Lanzettenvorrichtung offenbart, bei der ein Gewicht und eine in einer Bohrung des Gewichtes angeordnete Lanzette gemeinsam durch eine Feder beschleunigt werden. Gewicht und Lanzette treffen gemeinsam auf die Körperoberfläche zur Blutentnahme auf und die Lanzette bewegt sich aufgrund ihrer Massenträgheit innerhalb der Walze weiter, so daß sie in das darunterliegende Gewebe einsticht. In der Patentschrift ist beschrieben, daß durch die Walze ein Strecken der Haut erfolgt, welches die Haut stabilisiert und so den Einstich schmerzärmer macht. Dies wird dadurch erzielt, daß durch die Walze die Bewegungsfähigkeit der Haut im Bereich der Einstichstelle verringert wird. Die beschriebene Vorrichtung besitzt in der praktischen Anwendung jedoch eine Reihe von signifikanten Nachteilen. Wie aus Figur 5 der US 6,306,152 zu erkennen ist, ist die maximale Einstichtiefe der Lanzette durch die Länge einer Bohrung in der Walze gegeben, in der der obere Teil der Nadel läuft. Wegen Vorgabe der Einstichtiefe durch die Länge der Bohrung ist es nicht möglich, daß der Benutzer eine Regulierung der Einstichtiefe vornimmt, um für seine konkrete Hautbeschaffenheit eine günstige Stichtiefe wählen zu können, bei der mit minimalem Schmerz noch eine ausreichende Menge Blut für eine Analyse gewonnen wird. Die Bewegung der Lanzette relativ zur Walze durch reine Massenträgheit, wirft noch weitere, schwerwiegende Probleme auf. Zum einen ist es möglich, daß bei einer gegebenen Anordnung die zur Verfügung stehende Einstichtiefe nicht vollständig ausgenutzt wird, weil die Beschleunigung zu gering ist um in die Haut einzudringen. Erschwerend kommt hinzu, daß es produktionstechnisch schwierig ist, die Reibung zwischen der Lanzette und der Walze hinreichend zu kontrollieren. Schlimmstenfalls kann bei Produktionsschwankungen oder auch schon bei einer geringfügigen Verbiegung der Lanzette ein Verklemmen erfolgen, so daß die Massenträgheit nicht mehr ausreicht, überhaupt einen Stich durchzuführen. Ein weiterer Nachteil besteht darin, daß für einen Austausch der Lanzette, welcher aus hygienischen Gründen nach jedem Stich erfolgen sollte, auch ein Ausbau der Walze notwendig ist und die Lanzette aus dieser entnommen werden muß oder aber die gesamte Einheit aus Walze und Lanzette ausgetauscht werden muß.

Die Aufgabe der vorliegenden Erfindung bestand darin, eine Lanzettenvorrichtung zu Blutentnahme vorzuschlagen, welche den Schmerz bei der Entnahme minimiert und die eine hygienische Benutzung ermöglicht.

Gemäß der angegebenen Aufgabenstellung wird ein Lanzettensystem zur schmerzarmen Blutentnahme vorgeschlagen, welches ein Gehäuse mit einer Kontaktfläche aufweist, aus der die Spitze einer Lanzette austreten kann, wobei die in einem Lanzettenhalter befindliche Lanzette mittels eines Lanzettenantriebes verfahren wird und ein Impulsgeber zeitlich abgestimmt auf den Einstich der Lanzette einen Impuls auf ein Körperteil ausübt, so daß das durch den Einstich verursachte Schmerzempfinden verringert wird.

Ein Verfahren zur schmerzarmen Blutentnahme wird weiterhin beschrieben, bei dem in zeitlich abgestimmter Folge zum Einstich in eine Körperoberfläche ein Impuls auf ein Körperteil ausgeübt wird.

Durch das erfindungsgemäße System werden wesentliche Verbesserungen in dem Ziel erreicht, das Schmerzempfinden bei dem Stich mit einer Lanzette zu verringern. Durch die Bewegung des Lanzettenhalters und somit auch der Lanzette entlang eines vorbestimmten Einstichweges kann die Einstechtiefe kontrolliert und ggf. auch reguliert werden. Weiterhin wird durch eine zeitliche Abstimmung des auf die Haut ausgeübten Impulses relativ zum Zeitpunkt des Einstiches in den Körper eine Optimierung des Effekts der Schmerzmaskierung erzielt.

Das Lanzettensystem besitzt ein Gehäuse mit einer Kontaktfläche, die ein Benutzer des Systems auf einer Körperstelle positionieren kann, aus der eine Blutentnahme erfolgen soll. Das Gehäuse dient dabei sowohl zur Handhabung durch den Benutzer als auch zum Schutz der Mechanik und der Lanzette gegenüber der Umgebung. Die Kontaktfläche, welche an den Körper angedrückt wird, weist eine Öffnung auf, durch die die Spitze einer Lanzette aus dem Innenraum des Gehäuses austreten kann, um in Haut einzustechen. Die Austrittsöffnung in der Kontaktfläche weist vorzugsweise einen Querschnitt unter 5 mm, günstigenfalls zwischen 1 und 3 mm auf. Die Kontaktfläche, welche für eine Blutentnahme an den Körper angedrückt wird, kann beispielsweise die Form eines planen Kreisringes aufweisen. Es hat sich jedoch aus vorteilhaft erwiesen, für eine Blutentnahme aus der Fingerbeere auf der Kontaktfläche in direkter Nachbarschaft zu der Austrittsöffnung einen kleinen Wulst vorzusehen, der beispielsweise die Form eines um die Austrittsöffnung umlaufenden Ringes aufweist, um die Haut im Einstichbereich vorzuspannen. Weiterhin können an der Kontaktfläche auch Vorsprünge vorgesehen werden, die über die Ebene der Kontaktfläche hinausragen, und so beim Andrücken an den Körper eine Stimulierung der Nerven verursachen. Eine geeignete Anordnung ist in WO 01/62150 beschrieben. Die Kontaktfläche kann auch eine Kontur bzw. Wölbung aufweisen, die ein Austreten von Körperflüssigkeit aus der erzeugten Körperöffnung unterstützt. Eine derartige Kontaktfläche, die als Stimulatorfläche bezeichnet wird, ist in der WO 99/26539 beschrieben. Die Kontaktfläche kann zur Unterstützung einer Entnahme von Körperflüssigkeit auch deformierbar ausgestaltet sein, so daß ein Andrücken der Kontaktfläche an die Körperoberfläche eine laterale Bewegung erzeugt, welche einen Melkeffekt zur Folge hat. Eine derartige Kontaktfläche ist in der internationalen Anmeldung WO 01/89383 beschrieben, auf welche hiermit Bezug genommen wird.

Im Bereich der Blutentnahmesysteme hat es sich eingebürgert, austauschbare Lanzetten zu verwenden, die der Benutzer aus hygienischen Gründen nach einmaliger oder unter Umständen mehrfacher Benutzung austauschen kann. Insbesondere, wenn ein Blutentnahmesystem von mehreren Benutzern verwendet wird, ist eine leichte Austauschbarkeit der Lanzetten wichtig. Bei herkömmlichen Systemen erfolgt der Austausch beispielsweise durch Abnehmen einer Kappe am vorderen Ende des Gehäuses und Auswerfen der Lanzette aus einer Halterung. Es ist jedoch auch möglich, daß das System ein Magazin für Lanzetten aufweist, in das eine gebrauchte Lanzette zurückgezogen und / oder durch das eine neue Lanzette in den Lanzettenhalter eingebracht wird.

Unter einer Lanzette soll im Rahmen dieser Erfindung eine Vorrichtung verstanden werden, die eine Nadel mit einer Spitze aufweist, welche geeignet ist, eine Körperöffnung zu erzeugen. Eine solche Nadel kann dabei sowohl eine Vollnadel sein als auch eine Hohlnadel. Geeignete Materialien für die Nadel sind insbesondere Metalle, wie vor allem Edelstähle aber auch Klingen aus Flachstahl, Silizium, Keramik etc. Es ist möglich, eine solche Nadel ohne weiteres in einer Halterung des Blutentnahmesystems zu haltern. Vorteilhaft weist die Lanzette jedoch neben der Nadel einen Haltekörper auf, in dem sich die Nadel befindet. Solche Haltekörper können aus Kunststoff gefertigt sein, welcher in einem Spritzgußverfahren um die Nadel gespritzt wird. Durch diesen Haltekörper ist es einfach möglich, die Lanzette in einer Halterung des Systems zu haltern. Weiterhin kann der Haltekörper auch so ausgestaltet sein, daß er den Spitzenbereich der Nadel mit umschließt und ihn so vor Benutzung vor einer Kontamination schützt. Eine derartige Versiegelung kann durch Abdrehen an einer Sollbruchstelle entfernt werden, so daß die Nadelspitze für eine Blutentnahme frei liegt. Da Konzepte zur Halterung einer Nadel in einem Körper und auch die hygienische Versiegelung der Nadelspitze im Stand der Technik hinlänglich bekannt sind, wird an dieser Stelle nicht näher darauf eingegangen.

Zur Halterung der Lanzette besitzt das Blutentnahmesystem einen beweglichen Lanzettenhalter, der entlang eines vorbestimmten Weges verfahren werden kann, um einen Stich für eine Blutentnahme durchzuführen. Die Lanzette kann in dem Lanzettenhalter beispielsweise durch Preßsitz oder durch Verklemmen oder Verrasten gehaltert sein. Ein im Rahmen der vorliegenden Erfindung besonders geeigneter Lanzettenhalter ist in der EP 0 565 970 beschrieben. Der Lanzettenhalter muß jedoch nicht unbedingt ein Körper sein, der die Lanzette in einem Haltebereich umschließt, sondern es kann auch beispielsweise eine Art Dorn sein, auf den die Lanzette aufgebracht wird. Geeignete Kombinationen von Lanzetten und Lanzettenhaltern, die eine formschlüssige Ankopplung ermöglichen, sind in der WO 02/36010 beschrieben. Derartige Ankopplungsmethoden sind im Rahmen der vorliegenden Anmeldung besonders günstig, da aufgrund der formschlüssigen Verbindung sowohl der Hinweg der Lanzette zum Einstich als auch das Zurückziehen ins Gehäuse mit einer geführten Bewegung erfolgt, die sich als schmerzarm erwiesen hat.

Der Lanzettenhalter wird durch einen Lanzettenantrieb entlang eines vorbestimmten Weges bewegt, so daß eine Lanzette, die in dem Lanzettenhalter angeordnet ist, zeitweise durch die Austrittsöffnung austritt und in eine dort angeordnete Körperpartie einstechen kann. Im Stand der Technik sind eine Vielzahl von Lanzettenantrieben bekannt. Zum einen gibt es Antriebe, bei denen eine Antriebsfeder den Lanzettenhalter mit der Lanzette auf einen Anschlag fährt, wobei der Einstich in den Körper im Endbereich dieser Bewegung liegt. Die Feder kann so beschaffen sein, daß sie die Lanzette aus der Einstichposition wieder zurückzieht und eine Ruheposition einnimmt, bei der sich die Nadel innerhalb des Gehäuses befindet. Zum Zurückfahren der Nadel in das Gehäuse kann jedoch auch eine zweite Feder vorgesehen werden, die die Lanzette aus der Einstichposition in das Gehäuse zurücktreibt. Weiterhin kommen als Antrieb zwangs führende Systeme in Frage wie z. B. Kurbeltriebe, Hebelgetriebe oder Nockensteuerungen, bei denen die Bewegung der Lanzette nicht dem freien Spiel von Feder- und Massenkräften überlassen wird, sondern durch die Antriebsmechanik genau vorgegeben ist. Als besonders vorteilhaft hat sich im Rahmen der vorliegenden Erfindung ein Antrieb gemäß der EP 0 565 970 erwiesen, bei dem der Lanzettenhalter durch ein Drehschiebergetriebe verfahren wird. Der Lanzettenantrieb besitzt hierzu eine Hülse mit einer Nut, in der ein Steuerzapfen läuft, der mit dem Lanzettenhalter verbunden ist. Bei Drehung der Hülse um eine Achse parallel zur Einstichrichtung bewegt sich der Zapfen innerhalb der Nut und die Bewegung der Lanzette ist in axialer Richtung über die Formgebung der Nut im Rahmen von Toleranzen exakt bestimmbar. Durch die Führung des Zapfens in der Nut ist während der Bewegung eine exakte mechanische Führung der Lanzette sowohl in positiver als auch in negativer Einstichrichtung gegeben. Mit dieser, als positive Antriebskopplung bezeichneten Führung ist es möglich, definierte Weg- Zeit-Verläufe der Einstichbewegung zu generieren, die sich als besonders schmerzarm erwiesen haben.

Ein spezielles Merkmal des erfindungsgemäßen Blutentnahmesystems ist ein Impulsgeber, mit dem in zeitlich abgestimmter Abfolge zum Stechen mit der Lanzette ein Impuls auf ein Körperteil ausgeübt wird. Durch einen solchen Impuls werden Mechanorezeptoren im Gewebe gereizt. Diese Rezeptoren sind mit dicken Nervenfasern verbunden, die eine hohe Leitungsgeschwindigkeit haben. Schmerzrezeptoren (Nozizeptoren), welche durch den Einstich aktiviert werden, sind hingegen mit dünneren und langsamer leitenden Nervenfasern verbunden. Das Rückenmark stellt ein Gate dar, bei dem die Fasern aus der Peripherie ankommen und verschaltet werden. Die dünnen Nervenfasern von den Nozizeptoren öffnen das Gate, so daß Schmerzempfindungen zum Gehirn gelangen können. Die dicken Nervenfasern der Mechanorezeptoren hingegen schließen das Gate. Die Bilanz aus ankommenden Signalen von dünnen und dicken Fasern bestimmt das Schmerzempfinden. Diese Effekte sind auch unter der Bezeichnung Gate-Control-Theorie bekannt.

Durch einen mechanischen Impuls, der in zeitlich abgestimmter Folge zum Stich der Lanzette ausgeübt wird, kann durch Anregung der Mechanorezeptoren das Schmerzempfinden aufgrund eines Einstiches der Nadel reduziert oder ganz unterdrückt werden. Da die Signale der Mechanorezeptoren über die dicken Nervenfasern schneller geleitet werden als die Signale der Nozizeptoren, können sie, selbst wenn sie nach den Nozizeptoren gereizt werden, deren Signale überholen und so das Gate schließen, bevor das Schmerzsignal dort eintrifft. Erfindungsgemäß kann daher der Impuls auf das Körperteil sowohl vor dem Stich als auch noch kurz danach ausgeübt werden. Unsere Untersuchungen haben gezeigt, daß der Impuls, wenn er zwischen 1000 und 0 Millisekunden vor dem Stich oder zwischen 0 und 100 Millisekunden noch besser, zwischen 20 und 50 Millisekunden nach dem Stich ausgeübt wird zu einer Reduktion des empfundenen Schmerzes führt.

Der Impuls auf den Körperteil wird von dem Impulsgeber durch die Kontaktfläche des Gehäuses ausgeübt. Impulsgeber und Lanzettenantrieb können beispielsweise separat voneinander aufgebaut sein und von einer Steuervorrichtung elektrisch angesteuert werden. Es hat sich jedoch als vorteilhaft erwiesen, Impulsgeber und Lanzettenantrieb mechanisch miteinander zu koppeln, so daß der Impuls und der Stich durch diese mechanische Kopplung aufeinander abgestimmt sind. Spezielle Ausführungsformen mechanischer Kopplung werden im Zusammenhang mit den Figuren detaillierter beschrieben.

Bei Blutentnahmesystemen handelt es sich im Regelfall um relativ kleine Vorrichtungen, beispielsweise in Form eines Stiftes, die manuell gehaltert werden. Bei einer solchen Vorrichtung wird der Impuls durch eine Relativbewegung von begrenzt großen Massen. Das Verhältnis der Massen zueinander und ihre Relativgeschwindigkeit bestimmt und damit die auf das Körperteil ausgeübte Kraft zur Reizung der Rezeptoren. Es hat sich als vorteilhaft erwiesen, im Inneren des Gehäuses eine Masse zur Erzeugung des Impulses vorzusehen, die relativ zum Gehäuse bewegt wird. Wird diese Masse durch einen Antrieb von der Kontaktfläche wegbeschleunigt, so übt das Gehäuse mit der Kontaktfläche einen Impuls auf das Körperteil aus. Im Falle einer Irnpulsübertragung auf dem Körper mit Hilfe eines Stößels findet ebenfalls eine Relativbewegung von Massen statt, wobei sich nunmehr das Gehäuse vom Körperteil wegbewegt.

Erfindungsgemäß wurde gefunden, daß der Impuls vorzugsweise eine Dauer im Bereich von 0 bis 10 oder noch besser im Bereich zwischen 1 und 7 msec aufweisen sollte, um eine effiziente Schmerzmaskierung zu erzielen. Dies bedeutet, daß der Impuls nicht andauert sondern - vorzugsweise durch das Blutentnahmesystem selbst - nach kurzer Zeit wieder rückgängig gemacht wird, indem die auseinander bewegten Massen wieder zusammengezogen werden.

Die Kraft, die durch den Impulsgeber auf das Körperteil ausgeübt wird, liegt vorzugsweise im Bereich von 10 bis 30 N.

Die Erfindung wird nunmehr an einigen Beispielen näher erläutert:
Figur 1: Blutentnahmesystem mit einem Stößel als Impulsgeber
Figur 2: Weg-Zeit-Verläufe von Stich und Impuls mit dem in Figur 1 dargestellten System
Figur 3: Bewertung des Schmerzempfindens bei den in Figur 2 dargestellten Weg-Zeit-Verläufen durch Probanden
Figur 4: Blutentnahmesystem mit einem Drehschiebergetriebe
Figur 5: Blutentnahmesystem mit einer federgetriebenen Masse

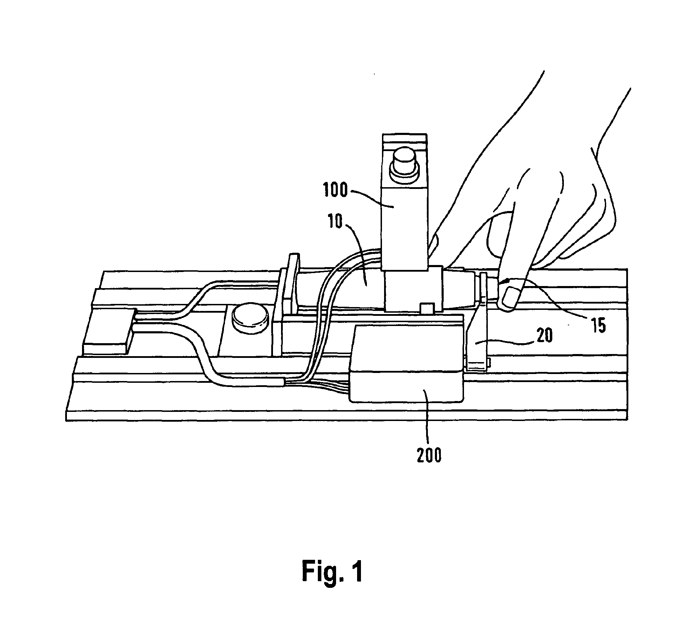

Figur 1 zeigt einen Testaufbau für ein Blutentnahmesystem mit einem Impulsgeber. Das System beinhaltet ein konventionelles Blutentnahmesystem (10), wie es in der EP 0 565 970 beschrieben und im Handel unter der Bezeichnung Softclix® erhältlich ist. Wie dargestellt, drückt ein Benutzer seine Fingerkuppe an die Kontaktfläche (15) aus der beim Stich eine Lanzettennadel austritt. Das Blutentnahmesystem besitzt einen Aktivierungsknopf, der durch einen elektromechanischen Aktuator (100) ausgelöst werden kann. Im Bereich der Kontaktfläche befindet sich ein Stößel (20), der durch einen zweiten elektromechanischen Aktuator (200) in Richtung auf das Körperteil verfahren werden kann. Erster und zweiter Aktuator sind mit einer elektrischen Steuereinheit (nicht dargestellt) verbunden, die die Aktuatoren in zeitlich abgestimmter Folge aktiviert. Mit der in Figur 1 dargestellten Anordnung wurden verschiedene Abfolgen von Stechen mit dem Blutentnahmesystem und einem Schlag mit der Gabel (20) untersucht.

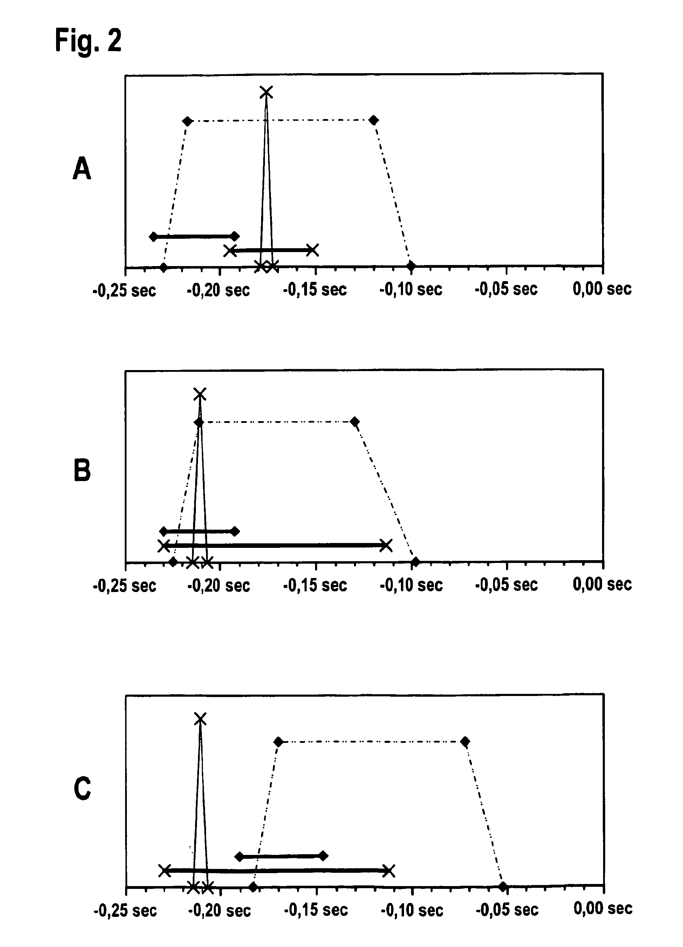

In Figur 2 sind die zeitlichen Abfolgen dargestellt. Gestrichelt dargestellte Kurven geben den zeitlichen Wegverlauf der Gabel (20) wieder. Die dünne durchgezogene Linie zeigt die zeitliche Bewegung der Lanzette. Die breiten waagerechten Linien geben an, in welchen Zeiträumen die Aktuatoren (100, 200) angesteuert wurden. Der erste Aktuator (100) für das Auslösen der Blutlanzette besitzt an seinen Enden Kreuze, während die Aktivierungsphase des zweiten Aktuators (200) für die Gabel durch Rauten dargestellt ist. Figur 2A zeigt eine Situation, bei der mit der Gabel ein Schlag ausgelöst und noch während sich die Gabel in Schlagposition befindet, der Stich ausgeführt wird. Gemäß Figur 2B wurde gestochen, während die Gabel beginnt, den Schlag auszuführen. In Figur 2C ist hingegen eine Situation dargestellt, bei der erst der Stich erfolgt und etwa 0,03 sec später der Schlag beginnt.

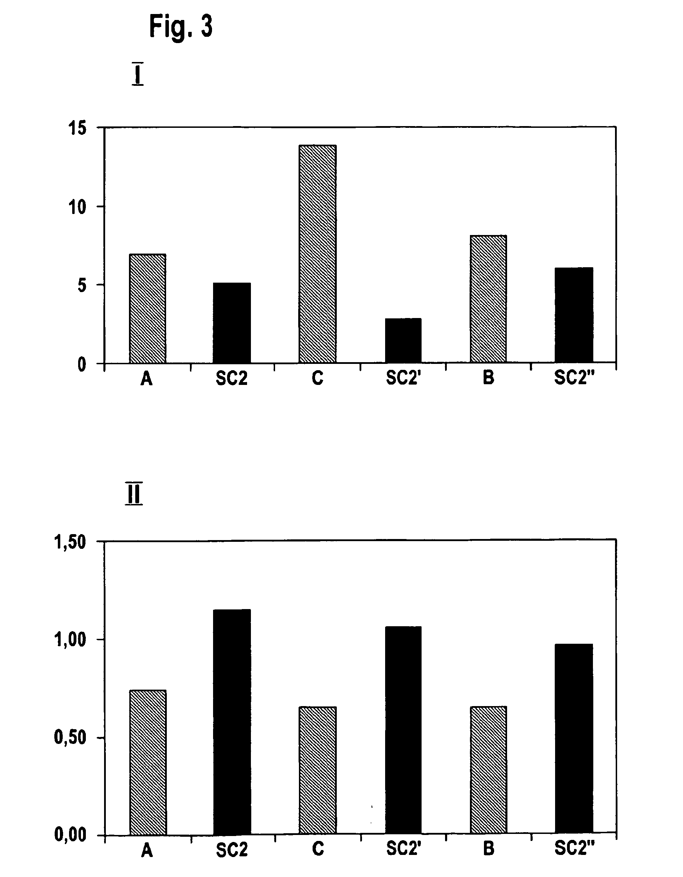

Figur 3 zeigt eine Untersuchung der bei den verschiedenen zeitlichen Abfolgen auftretenden Schmerzempfindungen. Die Versuche wurden mit 27 Versuchspersonen durchgeführt und mit entsprechenden Blutentnahmen bei den gleichen Probanden mit einem Softclix® verglichen. Figur 3I zeigt die Zahl der Blutentnahmen, die von den Probanden als schmerzfrei empfunden wurden. Die gestrichelten Balken entsprechen dabei den zeitlichen Abfolgen A, B, C wie in Figur 2 dargestellt. Die jeweils direkt auf der rechten Seite benachbarten schwarzen Balken zeigen die Zahl der als schmerzfrei empfundenen Blutentnahmen mit einem herkömmlichen Softclix®. Es ist aus der Figur zu entnehmen, daß die Zahl der als schmerzfrei empfundenen Blutentnahmen durch Ausüben des Impulses erhöht werden konnte. Insbesondere Variante C, bei der der Schlag ca. 30 msec nach dem Stich erfolgte, ist im Bezug auf das Schmerzempfinden günstig.

Figur 3II zeigt eine Einordnung der Schmerzhaftigkeit der Blutentnahmen mit und ohne Schlag. Die gestrichelt dargestellten Balken geben gemittelte Schmerzzuordnungen wieder, wie sie für die entsprechenden zeitlichen Abfolgen A, B, C aus Figur 2 erhalten wurden. Die rechts benachbarten schwarzen Balken stellen wiederum Vergleichsmessungen mit einem herkömmlichen Softclix® dar. Es ist auch in dieser Darstellung zu erkennen, daß durch die Verwendung des Impulses im Mittel eine deutliche Verringerung des empfundenen Schmerzes erzielt werden konnte.

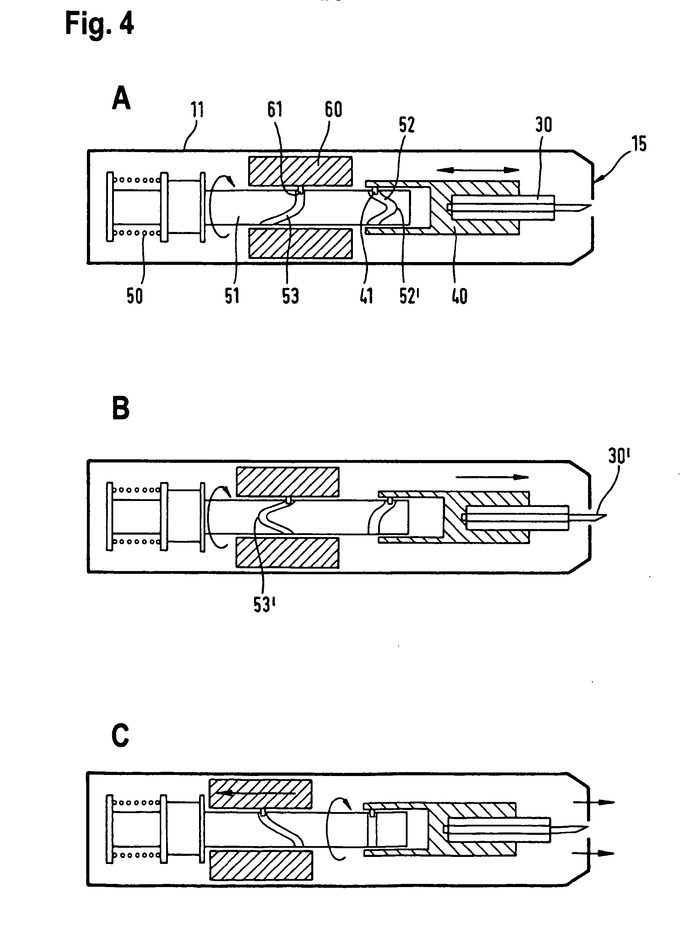

Figur 4 zeigt ein bahngesteuertes System mit einem Drehschiebergetriebe, das auf dem Antriebskonzept der EP 0 565 970 aufbaut. Das System besitzt ein Gehäuse (11), das zur manuellen Handhabung dient, und in dem die Antriebsmechanik angeordnet ist. Der Antrieb beinhaltet ein elastisches Antriebselement, im vorliegenden Fall eine Feder (50), die an einem Ende gegen das Gehäuse fixiert ist und an ihrem anderen Ende mit einem Getriebeglied, im speziellen Fall einer Führungshülse (51), verbunden ist. Durch Drehen der Führungshülse (51) kann die Feder (50) gespannt werden, so daß sie bei Entspannen der Feder in der in Figur 4A dargestellten Richtung rotiert. Die Hülse (51) trägt zwei Nuten (52, 53), von denen die erste (52) zum Antrieb des Lanzettenhalters (40) dient. In der Sequenz A,B,C ist dargestellt, wie der Lanzettenhalter und somit auch die in ihm fixierte Lanzette (30) bei Drehung der Hülse (51) verfahren wird. Hierzu besitzt der Lanzettenhalter (40) einen an ihm befestigten Zapfen (41), der in der ersten Nut (52) läuft. Der Lanzettenhalter ist gegen Rotation gesichert, so daß er bei Rotation der Hülse von Position A zu Position B durch den Zapfen nach vorn verschoben wird und zu dem Extrempunkt (52') gelangt. Hierbei tritt die Nadel (30') der Lanzette (30) durch eine Öffnung in der Kontaktfläche (15) aus dem Gehäuse aus und sticht ggf. in ein dort angedrücktes Körperteil. Während dieser Drehung der Hülse (51), die etwa 45° beträgt, durchläuft der Zapfen (61), welcher mit einer zylindrischen Masse (60) als Impulsgewicht verbunden ist, in der zweiten Nut (53) durch einen im wesentlichen geraden Bereich. Dies bedeutet, daß während der Drehung der Hülse von Figur 4A nach 4B nur eine geringfügige Beschleunigung der Masse (60) erfolgt. In der darauffolgenden Drehung von Figur B nach Figur C wird der Lanzettenhalter zurückbewegt, so daß die Lanzettenspitze ins Gehäuse gelangt. Während dieses Zurückziehens erfährt die Masse eine scharfe Änderung der Bewegungsgeschwindigkeit nach Betrag und/oder Richtung, indem der Zapfen (61) in die Schlagposition (53') gelangt. Durch diese relative Beschleunigung der Masse (60) und gegenüber der Masse, die durch die übrigen Systembestandteile gegeben ist, erfährt das Gehäuse einen Impuls in Richtung der Kontaktfläche, so daß diese auf ein an der Kontaktfläche befindliches Körperteil einen Schlag ausübt. Aufgrund der Funktionsweise ist ersichtlich, daß durch die radiale Position der Extrempunkte (52') und (53'), sowie die Rotationsgeschwindigkeit der Hülse (51) der zeitliche Abstand zwischen dem Stich und dem Schlag eingestellt werden kann. Durch die als Steuerkurven fungierenden Nuten (52, 53) kann der Weg-Zeit-Verlauf sowohl des Stiches und der Rückholbewegung als auch der zeitliche Verlauf des Schlages / Impulses exakt vorgegeben werden.

Bei diesem System ist es vorteilhaft, wenn einerseits die Masse des Antriebsmechanismus und des Gehäuses relativ klein sind, die Masse des Impulsgewichtes andererseits dagegen möglichst groß ist, damit die innere Kraft, die die beiden Massen relativ zueinander beschleunigt, zu einer möglichst großen Beschleunigung des Gerätegehäuses führt. Wäre das Impulsgewicht klein im Vergleich zu der Masse von Gehäuse und Antrieb so wäre diese Relativbeschleunigung gering.

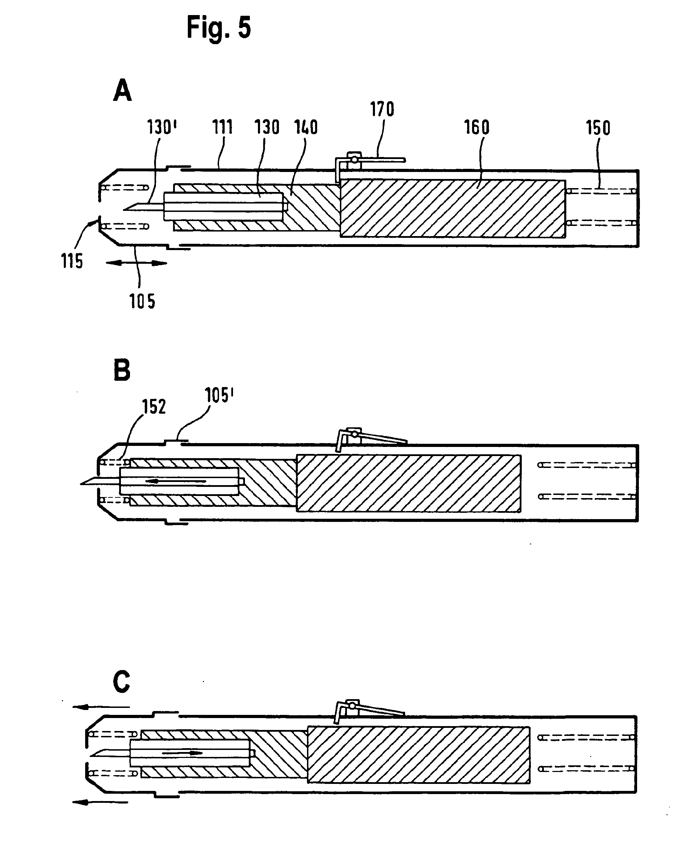

Figur 5 zeigt ein Blutentnahmesystem mit einem Impulsgeber, das einen Antrieb mit zwei elastischen Antriebselementen aufweist. In dem Gehäuse (111) ist eine Einheit aus einem Lanzettenhalter (140) und einer an ihm fixierten Masse (160), der Impulsmasse, angeordnet. Diese Einheit ist gegen eine Antriebsfeder (150) gespannt und mit einem Hebel (170) arretiert. Innerhalb des Lanzettenhalters befindet sich eine Lanzette (130) mit einer Nadel (130') zum Erzeugen einer Hautöffnung. Beim Lösen der Arretierung (170) wird die Einheit aus Lanzettenhalter und Masse und somit auch die Lanzette durch die Antriebsfeder (150) in Richtung auf die Kappe (105) beschleunigt, an der sich die Kontaktfläche (115) befindet. Diese Vorwärtsbeschleunigung erfolgt vorzugsweise auf einem relativ langen Weg, um die Beschleunigung in dieser Phase vom Betrag gering zu halten. In der in Figur 5B dargestellten Endposition tritt die Nadel (130') aus einer Öffnung in der Kontaktfläche (115) aus, so daß sie in einem an die Kontaktfläche angedrückten Körperteil eine Hautöffnung erzeugen kann. Aus Figur 5B ist weiterhin zu erkennen, daß durch die Einheit aus Lanzettenhalter und Masse eine zweite Feder, die Rückstellfeder (152), ausgehend von ihrer Ruheposition in Figur 5A, zusammengepreßt wird. Durch diese Feder, und in einer bevorzugten Ausführungsform zusätzlich durch einen festen Anschlag in der Kappe, wird die Vorwärtsbewegung des Lanzettenhalters mit seiner Masse zum Stillstand gebracht. Dieser Bremsvorgang erfolgt auf einem relativ kurzen Weg, woraus eine vom Betrag große Beschleunigung resultiert. Anschließend wird der Lanzettenhalter durch die Rückstellfeder zurückgezogen und damit die Nadel aus der erzeugten Wunde. Gehäuse (111) und Kappe (105) sind axial zueinander verschiebbar angeordnet, indem die Kappe (105) einen Bereich mit vergrößertem Innenquerschnitt (105') aufweist, in den der vordere Teil des Gehäuses (111) eingesteckt ist. Durch diese Beweglichkeit der Kappe wird die Reaktionskraft der Bremsbeschleunigung auf die Kappe übertragen, was einen Schlag auf ein an der Kontaktfläche befindliches Körperteil ausübt. Alternativ kann die Kappe starr mit dem Gerätegehäuse verbunden sein. Aber durch die Beweglichkeit der Kappe wird die Masse des Gerätegehäuses zwar zum Auffangen der Startbeschleunigung genutzt, stört aber nicht bei der Übertragung der Bremsbeschleunigung auf die Kappe durch die bewegte Masse und damit auf den Körper.

Wie beim bahngesteuerten Antrieb gemäß Figur 4 bereits erwähnt, werden innere Masseverschiebungen nach außen um so wirksamer, je größer die Impulsmasse und je kleiner die Masse des restlichen Gerätes ist. Im Fall des Feder-Masse-Antriebes gemäß Figur 5 ist es hingegen von Vorteil, wenn die Gehäusemasse relativ groß ist. Beim Auslösen des Stiches wird die Impulsmasse zusammen mit dem Lanzettenhalter nach vorne beschleunigt. Soll diese Antriebsbeschleunigung nicht dazu führen, daß das Gerät von der zu stechenden Oberfläche abgehoben wird, so muß die nach außen spürbare Reaktion hinreichend klein sein. Das gelingt durch lange Beschleunigungswege und damit geringe Beschleunigungsbeträge und/oder durch eine große Gehäusemasse. In dem Moment wo der Schlag ausgeübt wird, soll dagegen die Bremsbeschleunigung des Lanzettenhalters mit seiner Impulsmasse möglichst ungedämpft auf die zu stechende Oberfläche übertragen werden, was eine geringe Gehäusemasse relativ zur Impulsmasse erfordert. Die Beweglichkeit der Kappe koppelt nun einen (leichten) Teil vom übrigen Gehäuse ab, auf den dann die Bremsbeschleunigung der Impulsmasse wirkt, so daß die beiden gegensätzlichen Ziele (kein Abheben von Körperoberfläche, möglichst starker Impuls auf Körperoberfläche) in einem Gerät erreicht werden können.

Zum Spannen der Anordnung wird vorzugsweise ein Mechanismus verwendet, der beim Zurückziehen des Lanzettenhalters in die gespannte Position gemäß Figur 5A auch die Kappe in die Ausgangsposition gemäß Figur 5A zurückzieht, so daß sie beim Auslösen erneut eine Bewegungsmöglichkeit in Richtung der Kontaktfläche besitzt.

In das System gemäß Figur 5 kann auch ein Mittel zur Regulierung der Einstechtiefe integriert werden. Dies kann beispielsweise durch eine zweiteilige Kappe (105) erreicht werden, die ein erstes Teil besitzt, welches den Bereich mit vergrößertem Innenquerschnitt (105') umfaßt und weiterhin ein Gewinde aufweist. Ein zweiter Teil beinhaltet die Andruckfläche (115) und weist ebenfalls ein Gewinde auf, welches zu dem ersten Gewinde paßt. Die beiden Teile werden nunmehr aufeinandergeschraubt und durch Verschrauben der Teile gegeneinander kann die Einstechtiefe reguliert werden. Um eine konstante Einstechtiefe zu erzielen, ist es ferner günstig, die in Figur 5A dargestellte Ausgangsposition, bei der der Bereich mit verbreitertem Innenquerschnitt weitestgehend durch das Gehäuse (111) ausgefüllt ist, durch einen Anschlag oder andere Mittel definiert vorzugeben ist.

## Patentansprüche

1. Lanzettensystem (10) zur schmerzarmen Blutentnahme, umfassend,
• ein Gehäuse (11, 111) mit einer Kontaktfläche (15, 115), die eine Austrittsöffnung für die Spitze einer Lanzette aufweist,
• einen in dem Gehäuse (11) entlang einem Einstichweg beweglichen Lanzettenhalter (40. 140) zur Halterung der Lanzette,
• einen Lanzettenantrieb zum Verfahren des Lanzettenhalters (40, 140) entlang des Einstichweges
• einen Impulsgeber (60) mit dem in zeitlich abgestimmter Abfolge zum Verfahren des Lanzettenhalters (40, 140) ein Impuls auf ein Körperteil ausübbar ist, so dass aufgrund des ausgeübten Impulses ein Schmerzempfinden reduziert oder unterdrückt wird, **dadurch gekennzeichnet, dass** der Impuls durch die Kontaktfläche (15, 115) auf den Körper übertragen wird.

2. System gemäß Anspruch 1, welches so beschaffen ist, dass der Impuls zwischen 0 und 100 ms vorzugsweise zwischen 20 und 50 ms ausübbar ist, nachdem die Spitze der Lanzette aus der Austrittsöffnung ausgetreten ist.

3. System gemäß Anspruch 1, welches so beschaffen ist, dass der Impuls zwischen 1000 und 0 ms ausübbar ist, bevor die Spitze der Lanzette aus der Austrittsöffnung ausgetreten ist.

4. System gemäß Anspruch 1, das eine Einrichtung zur Verstellung der Austrittslänge der Lanzettenspitze über die Kontaktfläche (15, 115) aufweist.

5. System gemäß Anspruch 1, bei dem der Impulsgeber (60, 160) und der Lanzettenantrieb mechanisch miteinander gekoppelt sind.

6. System gemäß Anspruch 1, bei dem der Impulsgeber (60, 160) ein Drehschiebergetriebe mit einem Getriebeglied beinhaltet, das eine zum Einstichweg parallele Drehachse aufweist und das durch ein elastisches Antriebselement antreibbar ist, und das System weiterhin eine Masse beinhaltet, deren Bewegung durch das Getriebeglied steuerbar ist.

7. System gemäß Anspruch 6, bei dem die Bewegung des Lanzettenhalters (40, 140) durch das Getriebeglied steuerbar ist.

8. System gemäß Anspruch 1, das eine gegenüber dem Gehäuse (11, 111) verschiebbare Kappe aufweist, an der sich die Kontaktfläche mit der Austrittsöffnung befindet.

9. System gemäß Anspruch 8, das ein erstes elastisches Antriebselement zum Verschieben des Lanzettenhalters (40, 140) in eine Einstichposition und ein zweites elastisches Antriebselement zum Zurückführen der Lanzettenspitze in das Gehäuse (11, 111) aufweist, wobei das zweite elastische Antriebselement gegen die bewegliche Kappe gelagert ist.

10. System gemäß Anspruch 1, bei dem der Impulsgeber (60, 160) eine maximale Kraft im Bereich von 10 und 30 N auf den Körperteil ausüben kann.

11. System gemäß Anspruch 1, bei dem die Kontaktfläche (15, 115) eine profilierte Oberfläche aufweist mit der der Impuls appliziert wird.

## Claims

1. Lancet system (10) for pain-reduced blood withdrawal comprising,
• a housing (11, 111) with a contact surface (15, 115) which has an exit opening for the tip of a lancet,
• a lancet holder (40, 140) for holding the lancet which can move in the housing (11) along a lancing path,
• a lancet drive for moving the lancet holder (40, 140) along the lancing path,
• an impulse generator (60) which can exert an impulse on a body part in a sequence that is synchronized with the movement of the lancet holder (40, 140) so that pain sensation is reduced or suppressed as a result of the exerted impulse, **characterized in that** the impulse is transferred by the contact surface (15, 115) onto the body.

2. System according to claim 1 which is designed such that the impulse can be exerted between 0 and 100 ms and preferably between 20 and 50 ms after the tip of the lancet has emerged from the exit opening.

3. System according to claim 1 which is designed such that the impulse can be exerted between 1000 and 0 ms before the tip of the lancet has emerged from the exit opening.

4. System according to claim 1 which has a device for adjusting the length of the lancet tip which protrudes beyond the contact surface (15, 115).

5. System according to claim 1 in which the impulse generator (60, 160) and the lancet drive are mechanically coupled together.

6. System according to claim 1 in which the impulse generator (60, 160) comprises a rotary slide gear with a gear member which has an axis of rotation which is parallel to the lancing path and which can be driven by an elastic drive element, and the system additionally comprises a mass whose movement is controlled by the gear member.

7. System according to claim 6 in which the movement of the lancet holder (40, 140) can be controlled by the gear member.

8. System according to claim 1 which has a cap that can be moved relative to the housing (11, 111), the contact surface with the exit opening being located on said cap.

9. System according to claim 8 which has a first elastic drive element for moving the lancet holder (40, 140) into a lancing position and a second elastic drive element for retracting the lancet tip into the housing (11, 111), wherein the second elastic drive element is mounted against the movable cap.

10. System according to claim 1 in which the impulse generator (60, 160) can exert a maximum force in the range of 10 to 30 N on the body part.

11. System according to claim 1 in which the contact surface (15, 115) has a profiled surface with which the impulse is applied.

## Revendications

1. Système de lancette (10) pour le prélèvement indolore du sang, comprenant :
• un logement (11, 111) comprenant une surface de contact (15, 115) qui présente une ouverture de sortie pour la pointe d'une lancette ;
• un support de lancette (40, 140) mobile dans le logement (11) le long d'une voie de piqûre, pour le maintien de la lancette ;
• une commande de lancette pour déplacer le support de lancette (40, 140) le long de la voie de piqûre ;
• un générateur d'impulsions (60) avec lequel, d'une manière coordonnée dans le temps, en vue du déplacement du support de lancette (40, 140), une impulsion est exercée sur une partie du corps, si bien que, sur base de l'impulsion exercée, on réduit ou on supprime une sensation de douleur, **caractérisé en ce que** l'impulsion est transmise au corps via la surface de contact (15, 115).

2. Système selon la revendication 1, qui est conçu de telle sorte que l'impulsion peut s'exercer dans un laps de temps entre 0 et 100 millisecondes, de préférence entre 20 et 50 millisecondes, après la sortie de la pointe de la lancette hors de l'ouverture de sortie.

3. Système selon la revendication 1, qui est conçu de telle sorte que l'impulsion peut s'exercer dans un laps de temps entre 1000 et 0 millisecondes avant la sortie de la pointe de la lancette hors de l'ouverture de sortie.

4. Système selon la revendication 1, qui présente un dispositif pour le réglage de la longueur de sortie de la pointe de la lancette au-delà de la surface de contact (15, 115).

5. Système selon la revendication 1, dans lequel, le générateur d'impulsions (60, 160) et la commande de lancette sont couplés l'un à l'autre par voie mécanique.

6. Système selon la revendication 1, dans lequel le générateur d'impulsions (60, 160) contient une transmission à tiroir rotatif, comprenant un membre de transmission qui présente un axe de rotation parallèle à la voie de piqûre et qui peut être entraîné via un élément d'entraînement élastique, le système comprenant en outre une masse dont le mouvement peut être commandé par le membre de transmission.

7. Système selon la revendication 6, dans lequel le mouvement du support de lancette (40, 140) peut être commandé par le membre de transmission.

8. Système selon la revendication 1, qui présente un capuchon apte à se déplacer par rapport au logement (11, 111), contre lequel se trouve la surface de contact qui comprend l'ouverture de sortie.

9. Système selon la revendication 8, qui présente un premier élément d'entraînement élastique pour le déplacement du support de lancette (40, 140) dans une position de piqûre et un deuxième élément d'entraînement élastique pour la rentrée de la pointe de lancette dans le logement (11, 111), le deuxième élément d'entraînement élastique étant monté contre le capuchon mobile.

10. Système selon la revendication 1, dans lequel le générateur d'impulsions (60, 160) peut exercer une force maximale dans la plage de 10 à 30 N sur la partie du corps.

11. Système selon la revendication 1, dans lequel la surface de contact (15, 115) présente une surface profilée avec laquelle on applique l'impulsion.
